Europäisches Patentamt

European Patent Office

Office européen des brevets

(19)

(11) Publication number: 0 392 475 A2

# (12) EUROPEAN PATENT APPLICATION

(21) Application number: 90106887.4

(22) Date of filing: 10.04.90

(51) Int. Cl.5: G01N 1/28, G01N 15/14, G01N 33/49, G01N 35/00

(30) Priority: 11.04.89 JP 92367/89

(43) Date of publication of application:
17.10.90 Bulletin 90/42

(84) Designated Contracting States:
BE CH DE FR GB IT LI NL SE

(71) Applicant: IDEMITSU PETROCHEMICAL CO. LTD.
1-1, Marunouchi 3-chome Chiyoda-ku
Tokyo 100(JP)

(72) Inventor: Yamaji, Kazutaka, c/o Idemitsu Petroch. Co. Ltd.
1660, Kamiizumi, Sodegaura-cho
Kimitsu-gun, Chiba-ken(JP)
Inventor: Takase, Minoru, c/o Idemitsu Petroch. Co. Ltd.
1660, Kamiizumi, Sodegaura-cho
Kimitsu-gun, Chiba-ken(JP)

(74) Representative: Hoeger, Stellrecht & Partner
Uhlandstrasse 14 c
D-7000 Stuttgart 1(DE)

(54) Analysis apparatus.

(57) Disclosed is an analysis apparatus for testing blood and other samples by a quantative analysis and a qualitative analysis, in which samples are developed on sample development surfaces (5) of a rotatable disk (1) by a centrifugal force due to a rotation of the disk, the developed samples are scanned by an optical measuring head (14), and a sample test is carried out by a sample analysis unit (26).

FIG. 1

## ANALYSIS APPARATUS

### BACKGROUND OF THE INVENTION

1.Field of the Invention

The present invention relates to an analysis apparatus for carrying out a qualitative analysis and a quantative analysis of blood or other samples.

2.Description of the Related Art

Recently, to enable an early diagnosis of disease, the number of items tested in medical institutions has been greatly increased. Among these tests is the blood test, in which a classification, a counting, and a shape examination of red blood cells, white blood cells, blood platelets and other blood components are carried out. Furthermore, to enable an early diagnosis of cancers or AIDS, hormone, virus, and immunity constituents tests are carried out. As is clear from the above, many items must be tested, and therefore, the development of an analysis apparatus by which tests can be carried out in a short time, a high accuracy, and automatically, has become urgent need.

Apparatuses for analyzing samples including blood are disclosed in Japanese Unexamined Patent Publication (kokai) No. (JPA) 55-42046, JPA 56-98635, Japanese Examined Patent Publication (kokoku) No. (JPB) 54-36879, JPA 61-193072 and ANALYTICAL LETTERS, 7, 591-597(1974). (hereinafter, referred to simply as ANALYTICAL LETTERS).

The analysis apparatuses disclosed in JPA 55-42046 and JPA 56-98635 process an image enlargement signal enlarged of images of blood as a test body for testing blood. The analysis apparatuses disclosed in JPB 54-36879 and JPA 61-193072 use a reactive test to cause a reaction with a liquid sample, held in a cavity of a rotatable disk, by which reactive products are extracted from the disk, and the reactive products measured by, for example, a colorimetric analysis. The analysis apparatus disclosed in ANALYTICAL LETTERS uses a reactive test to cause a reaction with a liquid sample held on a rotatable disk, and measures reactive products obtained at an outer edge of the disk.

In the analysis apparatuses disclosed in JPA 55-42046 and JPA 56-98635, since the test is carried out by using the image enlargement signal of the enlarged images, the test is limited to a partial testing, and thus a high testing accuracy can not be obtained, and further, continuous measurements can not be carried out. In the analysis apparatuses disclosed in JPB 54-36879 and JPA 61-193072, since the reactive products are extracted from the disk and measured, not all of the processes for the test can not be carried out on the disk, and thus the processes and the construction thereof become complex. The analysis apparatus disclosed in ANALYTICAL LETTERS is not affected by the disadvantages described above, because the reactive products are measured at the outer edge of the disk. But since this analysis apparatus is constructed to collect all reactive products at the outer edge of the disk, this analysis apparatus can not be adapted, for testing of immunity constituents, which requires separation and rinse processes, and can not be applied to a shape analysis.

### SUMMARY OF THE INVENTION

An object of the present invention is to provide an analysis apparatus by which all of the processes of a test can be carried out on a disk, samples spread over the whole surface of the disk can be measured, to thereby obtain a high testing accuracy, and which can test many different items.

An analysis apparatus according to the present invention includes a disk surface on which sample development (spreading) surfaces are provided, samples being developed on the sample development surfaces, a disk rotating means for rotating the disk, and an optical measuring head, movable radially across the disk, and comprising an sample analysis means which scans and analyses the samples developed on the sample development surfaces of the disk by a centrifugal force due to rotation of the disk, and a control means for controlling the disk rotating means and the sample analysis means.

The number of the sample development surfaces is sufficient by one, but preferably, it should provide a plurality of sample development surfaces divided into compartments along a circumference of of the disk.

An exposure light emitted from the optical measuring head to expose each of the samples which are rotated to use an analysis is a convergence light or a linear pattern light having a length in the radial direction of the disk.

Each of operations of the disk rotating means and the sample analysis means is carried out under the control of the control means, and accordingly, the samples provided on the disk are developed on the sample development surfaces by a centrifugal force due to the rotation of the disk, and thereafter, a measurement of the samples is made by the optical measuring head of the sample analysis means. This head is moved in the radial direction of the disk while the disk is rotated, so that the head scans the samples on the surfaces of the disk, and consequently, an analysis of the samples developed on the sample development surfaces is carried out.

As a result, all of the processes for the testing can be carried out on the disk, and a simplification of the overall structure of the apparatus and a compact size thereof can be achieved, and further, the efficiency of the tests is improved.

Also, if a plurality of the sample development surfaces are provided by dividing the surface of the disk into compartments in the circumference, since, in accordance with the rotation of the disk, a supply of samples to the respective sample development surfaces and a measurement of the respective samples supplied thereto can be carried out, and the testing for different samples can be simultaneously carried out.

· Furthermore, if the light from the optical measuring head to which the samples are exposed, for analysing the samples, is a linear pattern light having a length in the radial direction of the disk, a distance for which the optical measuring head is moved in the radial direction of the disk, to scan the sample, can be shortened, and thus the test operation time can be reduced.

## BRIEF DESCRIPTION OF THE DRAWINGS

Other objects and features of the present invention will be described below in detail with reference to the accompanying drawings, in which:

Fig. 1 is a view illustrating an apparatus of an embodiment according to the present invention, in which a disk is shown by a sectional view and constitutional components of a plurality of means of the present invention are shown by a block diagram;

Fig. 2a is a plane view of the disk shown in Fig. 1, wherein the projections thereon are linear projections;

Fig. 2b is a plane view of the disk shown in Fig. 1, wherein the projections thereon are curved projections;

Fig. 3 is a view representing a construction of an optical measuring head shown in Fig. 1;

Fig. 4 is a view representing another construction of an optical measuring head shown in Fig. 1;

Fig. 5a is another plane view of a disk similar to Fig. 2a, when a light emitted from the optical measuring head is a linear pattern light;

Fig. 5b is a view illustrating an arrangement of a (line) slit for exposing the linear pattern light;

Fig. 6 is a plane view of a disk used for immuneoassay similar to Fig. 2; and

Fig. 7 is a sectional view taken along the lines VII- VII in Fig. 6.

## DESCRIPTION OF THE PREFERRED EMBODIMENTS

Preferred embodiments of the present invention will be described with reference to the drawings.

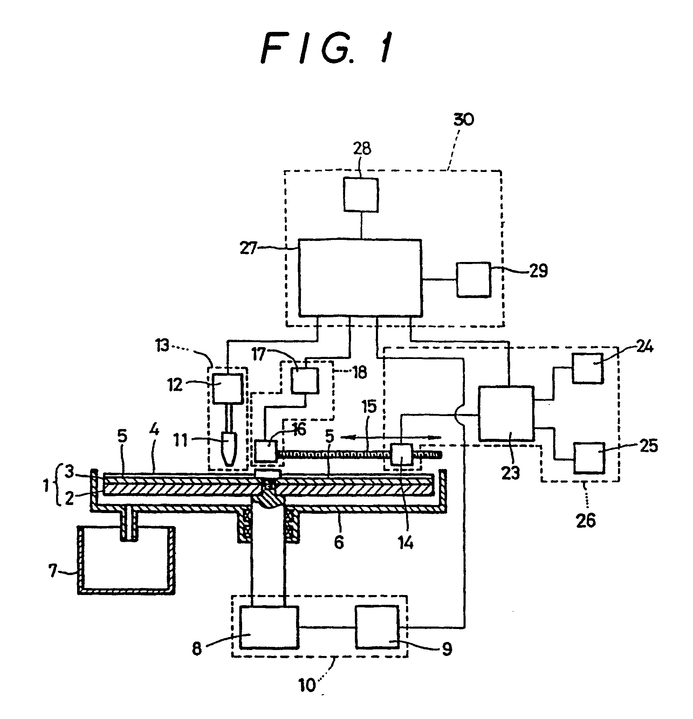

Figure 1 is a view illustrating a construction of an embodiment of an analysis apparatus according to the present invention. A sample which is tested by this analysis apparatus is a blood sample. A disk 1, to which the the blood sample is supplied, is positioned horizontally in a normal state and is constructed by a lower disk main body 2 and an upper test plate 3 made of a thin plate. This thin-plate like test plate 3 can be freely attached to and detached from the lower disk main body 2. The thin-plate like test plate 3 is formed , for example, of polycarbonate, polystyrene or polymethylemethacrylate, and is treated to improve the hydrophilic property of said disk(s) if required.

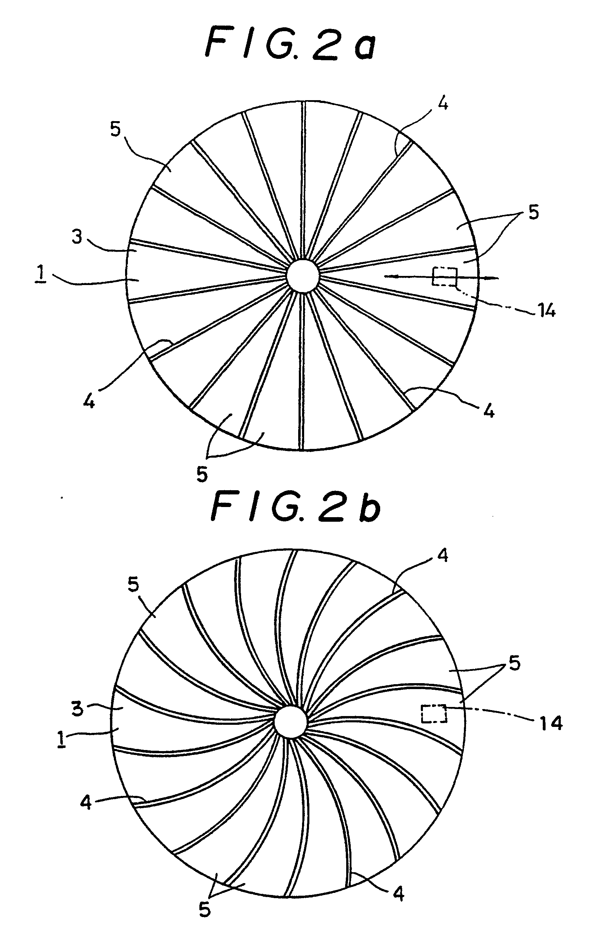

As shown in Fig. 2a, many protrusions 4 are formed radially on a horizontal plane of the test plate 3, and accordingly, many sample development (spreading) surfaces 5, which are divided into compartments along a circumference of the test plate 3, are provided on the surface of the test plate 3.

A container 6, which receives the sample dropped from the disk 1, is arranged below and at an outer circumference of the disk 1, to thereby enclose the disk 1, and samples received in the container 6 are thereafter collected in a collection tank 7.

A servometer 8, such as a pulse motor, is connected to the disk 1, and driven by a drive control circuit 9. A disk rotating means 10 for rotating the disk 1 is constituted by the servomotor 8 and the drive control circuit 9. An dispensing nozzle 11 is arranged above and adjacent to a center portion of the disk 1. The nozzle 11 is connected to a sample delivering device 12 which delivers all the blood samples to be tested to the disk 1, and/or if required, adds a diluent to the blood sample. A sample supply means 13 is constituted by the nozzle 11 and the sample delivering device 12.

An optical measuring head 14 is arranged above the disk 1 and secured to a delivering screw shaft 15 extending in a radial direction of the disk 1. The delivering screw shaft 15 is connected to a servomotor 16, such as a pulse motor, and the servomotor 16 is driven by a drive control circuit 17. The delivering screw shaft 15 is rotatated by the servomotor 16 clockwise or counterclockwise with respect to a center of the shaft, and consequently, the optical measuring head 14 is reciprocatively moved radially over the disk 1. A head moving means 18 by which he optical measuring head 14 is moved radially over the disk 1, is constructed by the servomotor 16, the drive control circuit 17, and the delivering screw shaft 15.

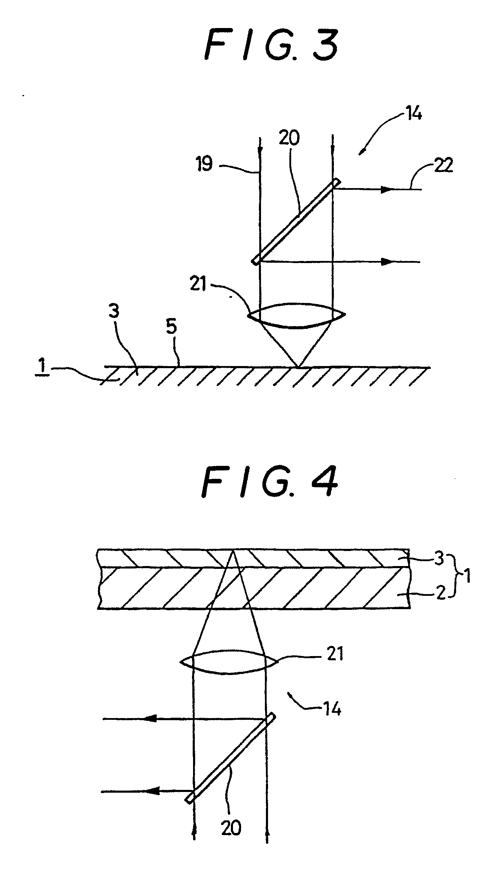

The optical measuring head 14 includes a light emitting portion (not shown) for emitting a laser light, and a light receiving portion (not shown). As shown in Fig. 3, an exposure light 19 emitted from the light emitting portion is passed through a half mirror 20 and an objective lens 21 become a covergent light, and the samples on the sample development surfaces 5 are exposed by the convergent light. A reflection light 22 reflected from the sample is received at the light receiving portion.

A signal processing apparatus 23 is connected to the optical measuring head 14, and functions to energise a light source of the light emitting portion in the head 14, on the basis of a predetermined input signal, to start a sample test, and further processes an information signal based on a light received at the light receiving portion in response to the test items, to carry out predetermined analyses. This information signal based on the light involves a change of a reflection index, an absorption to a light having a specific wave length, a fluoerscent intensity, and a rotational angle of a polarization plane of a polarized light, etc.

Alternatively, if a laser covergent light is used as the exposure light, information on each point of the samle development surfaces can be collected at a 1 $\mu$m order, and therefore, an analysis of the number of blood cells, etc. can be carried out without using enlarged images. More specifically, if the blood test involves a classification and. counting of red blood cells, the white blood cells and the blood platelets, the light received at the light receiving portion can be processed by a filter with respect to a wave length thereof, then, processed for forming images and converted to an electrical signal, and further processed the information signal obtained by the above processing, to carry out the classification and counting of the red blood cells, the white blood cells and the blood platelets.

A display unit 24 and a recording unit 25 are connected to the signal processing apparatus 23, to display analysed results obtained at the the signal processing apparatus 23 on a display panel of the display unit 24, and to print out the analysed results through the recording unit 25.

A sample analysis apparatus 26, which carries out required blood tests and outputs the test results as a display and/or a print-out, is constituted by the optical measuring head 14, the signal processing apparatus 23, the display unit 24, and the recording unit 25, as described above.

The drive control circuit 9 of the disk rotating means 10 described above, the sample delivering device 12 of the sample supply means 13 described above, the drive control circuit 17 of the head moving means 18, and the signal processing apparatus 23 of the sample analysis apparatus 26 are connected to a CPU (central processing unit) 27, respectively. By this CPU 27, the disk rotating means 10, the head moving means 18 and the sample analysis apparatus 26 are operated in accordance with programs previously set in a storage unit 29. More specifically, the control of the rotation speed of the disk rotating means 10 and of the head moving means 18, the timing controls for starting and stopping the rotation of the disk 1, and the control of operation timing of the sample supply means 13 and the sample analysis apparatus 26 are carried out in accordance with the programs, and the control necessary for automatically testing the blood is carried out by the CPU 27. The programs executed by the CPU 27 are set by an operation unit 28 and stored in the storage unit 29.

Control means 30 is comprised of the CPU 27, the operation unit 28, the storage unit 29, the disk rotating means 10, the sample supply means 13, the head moving means 18 and the sample analysis apparatus 26. And, the CPU 27, the operation unit 28 and the storage unit 29 control the disk rotating means 10, the sample supplying means 13, the head moving means 18 and the sample analysis apparatus 26 to carry out an automatic blood test.

In addition, a sample development surface detecting apparatus (not shown in the drawings), which reads an address code marked on each of the sample development surfaces 5 of the disk 1 and detects a corresponding sample development surface 5, can be connected to the CPU 27. According to this construction, a supply of a predetermined amount of blood to desired sample development surfaces 5 and a confirmation of the amount of blood held in predetermined sample development surfaces 5 by the sample analysis apparatus 26 can be carried out at the same time, as the automatic blood test is carried out.

The blood test carried out by the programs will now be described.

When a start switch provided on the operation unit 28 of the control means 30 is operated, a low speed rotation of the disk 1 is started by the disk rotating means 10. Simultaneously, all of the whole blood samples to be tested, which have been diluted by a diluent if required, are sequentially dropped from the nozzle 11 of the sample supply means 13 on the basis of the signal from the CPU 27, and accordingly, the blood as the samples are supplied to the respective sample development surfaces 5 of the disk 1. Thereafter, the rotation speed of the disk 1 is increased to a high speed, and as a result, the blood sample held on the sample development surfaces 5 are moved to outer circumferential portions of the disk 1 by a centrifugal force and developed (spread) on the sample development surfaces 5. Surplus blood samples are dropped into the receiving container 6, from the sample development surfaces 5, and collected in the collection tank 7.

In the embodiment of the present invention, the container 6 and the collection tank 7 are used, but a sample absorption portion formed by an absorptive material which absorbs surplus blood samples can be provided at the edges of the outer circumferential portions of the disk 1, instead of the above receiving container 6 and collection tank 7. According to this construction, a splashing of the samples can be prevented and a clean test can be achieved, accordingly. At the same time, the structure of the apparatus can also be simplified by the removing the receiving container 6 and the collection tank 7.

After the blood sample on the sample development surfaces 5 are developed, the rotation of the disk 1 is stopped, and after a predetermined time has elpsed, developed sample is dried and as a result, blood application specimens appear on the sample development surfaces 5.

Next, the blood testing is started by the sample analysis apparatus 26 in response to a signal from the CPU 27. This blood testing is carried out while the disk 1 is rotated at a low rotation speed and the optical measuring head 14 is moved radially over the disk 1 by the head moving means 18. Therefore, the optical measuring head 14 is reciprocatively moved a plurality of times for one sample development surface 5 radially over the disk 1. Consequently, the blood application specimens developed on the respective sample development surfaces 5, over a large area, are subjected to a multiple-scanning by the optical measuring head 14. An information signal obtained by the scanning by the head 14 is sent to the signal processing apparatus 23, an analysis of the blood sample is made at the signal processing apparatus 23. This blood analysis is carried out to judge the respective sample development surfaces 5, in response to the signal sent from the CPU 27 or the signal from the CPU 27, through the sample development detection apparatus described above. The results are displayed on the display panel of the display unit 24 and printed-out by the recording unit 25.

Upon completion of the operations described above, the operation of the disk rotating means 10, the sample supply means 13, the head moving means 18, and the sample analysis apparatus 26 are terminated.

When a subsequent blood test is required, the above-mentioned test plate 3, which is freely attached to and detached from the lower disk main body 2, is replaced by a new test plate 3, then, the start switch mounted on the operation unit 28 of the control means 30 is again operated, and the next blood test is started.

In the embodiment of the present invention, as described above, since the analysis apparatus is constructed to develop the blood sample on the sample development surfaces 5 by the centrifugal force obtained by he rotation of the disk 1 and, to scan the blood sample by moving the optical measuring head 14 of the samle analysis apparatus 26 radially over the disk 1, a testing over a wide area of the developed blood samples can be carried out. Note, this testing is not the testing by using the enlarged images of a small portion of the blood sample. As a result, a high test accuracy can be realized, and many different items can be tested at the same time.

In addition, all of the processes for carrying out the supply of the blood samples to the sample development surfaces 5 of the disk 1 by the sample supply means 13, the development of the blood samples over a wide development are on the sample development surfaces 5, and the analysis of the blood by the sample analysis apparatus 26, can be carried out on the disk 1, and accordingly, the apparatus can be given a simple and compact overall structure. Also, since the disk rotating means 10, the sample supply means 13, the head moving means 18, and the sample analysis apparatus 26 are respectively controlled by the control means 30, the blood testing can be carried out automatically, and accordingly, the working efficiency can be improved.

Furthermore, since a plurality of the sample development surfaces 5 are provided by radially dividing the surface of the disk 1 into compartments reaching from the center to the circumference thereof, the supply of the blood samples to the respective sample development surfaces 5 and the measurement of the respective samples supplied thereto can be carried out in accordance with the rotation of the disk, and therefore, many different samples can be simultaneuously tested.

In the above embodiment of the present invention, the projections 4 which radially divide the sample development surfaces 5 into compartments reaching from the center to the circumference of the disk 1 are linear, but as shown in Fig. 2b, the projections 4 may be formed by curvatures to enable a smoother development of the blood samples on the sample development surfaces 5 by the centrifufal force due to the rotation of the disk 1.

Also, although the blood testing processes for the blood tests, together with the moving of the optical measuring head 14 radially over the disk 1, can be carried out while the disk 1 is continuously rotated as described above, the blood testing processes may be carried out while intermittently rotating the disk 1.

The movement of the optical measuring head 14 radially over the disk 1 may be realized by an electrical means, for example, a linear motor, in addition to the mechanical means such as the delivering screw shaft 15 described above.

Figures 4 to 7 show constructions of another embodiment of an analysis apparatus in accordance with the present invention.

Figure 4 is a view illustrating an arrangement of the optical measuring head 14. In this embodiment, all of the structure of the disk 1 comprised of the lower disk main body 2 and the test plate 3 is formed of a transparent material, the optical measuring head 14 including the half mirror 20 and the (objective) lens 21 is arranged beneath the disk 1, and an exposure light from the light emitting portion is passed through the disk 1. In this embodiment, the exposure light is normally a polarized laser covergent light. Since this polarized laser covergent light enables an easy adjustment of a focus on the sample development surfaces, tests on a micron order can be easily carried out.

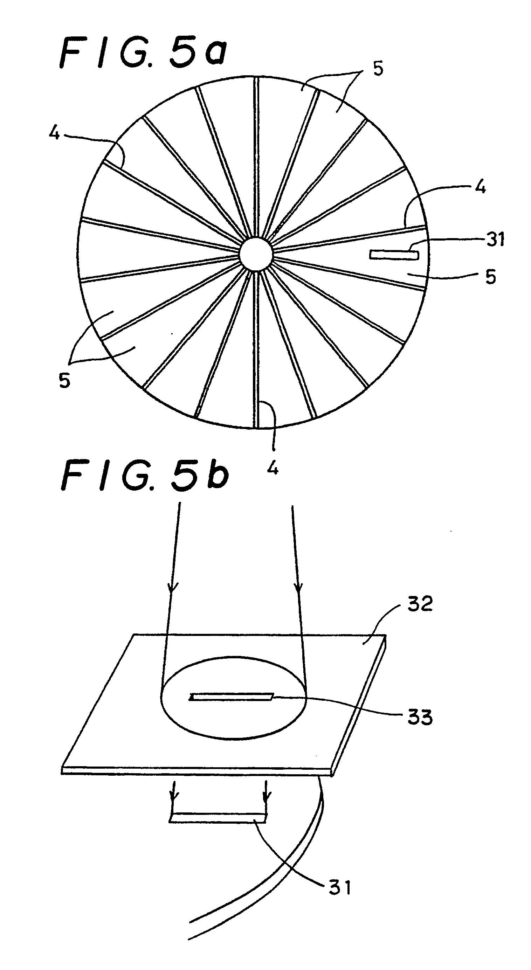

Figure 5a is another plane view of the disk 1, when a long linear pattern light emitted from the optical measuring head 14 is exposed on the sample development surfaces 5 of the disk 1. The exposure light emitted from the optical measuring head 14 was a convergent light in the embodiment previously described, however, in this embodiment, the exposure light is the long linear pattern light extending in the radial direction of the disk 1. To generate this long linear pattern light 31, as shown in Fig. 5b, a slit plate 32 having a narrow width slit 33 formed therethrough is arranged in a light path from the light emitting portion. In this embodiment, since the length of the long linear pattern light 31 lies in the radial direction of the disk 1, a head movement distance wherein the optical measuring head 14 is moved radially over the disk 1 to scan the blood samples can be shortened, resulting in a reduction of the time needed for the blood test.

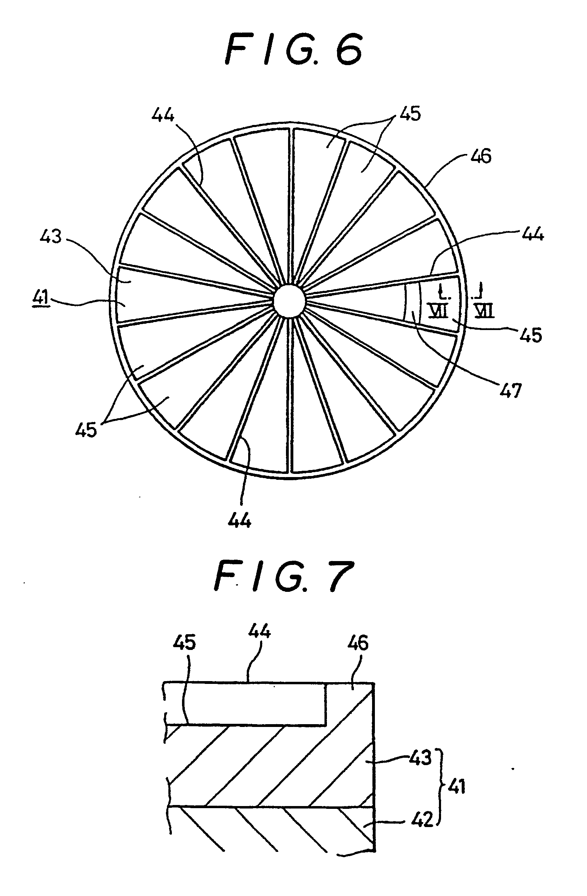

Figures 6 and 7 show still another embodiment of the present invention for an immunoassay of the blood based on antigen-antibody reaction. On a surface of an test plate 43, formed by a disk test plate 41 and a disk main body 42, radial projections 44 and an edge embankment 46 at an outer circumference of the disk 1 together formed compartmented sample development surfaces 45 provided radially on the test plate 43, from the center to the circumference thereof. Impregnation substances 47 having antibodies, which have the specific reaction sites with antigens, impregnated therein are arranged on the respective sample development surfaces 45. Blood sera with a definite amount of the cooresponding antigens labeled with fluorescent compounds are supplied to the sample development surfaces 45. These blood sera are transferred to the impregnation substances 47 due to the rotaion of the disk 41, and antitens in blood sera react with antibodies immobilized in impregnation substances 47 to form immuno-complexes. The antigens in the blood sera are analysed as fluorescent analysis data by so-called competitive immunoassay.

In the above embodiments of the present invention, blood is tested as the sample, but the analysis apparatus in accordance with the present invention is not limited to the testing of blood, but can be of course applied to the carrying out of a quanlative analysis and a quantative analysis of other kinds of samples.

## Claims

1. An analysis apparatus comprising a disk having a surface on which sample development surfaces are provided, and disk rotating means for rotating said disk,
characterized in that it comprises
an optical measuring head movable radially over said disk,
sample analysis means for scanning and analysing samples developed on the sample development surfaces of said disk by centrifugal force due to a rotation of said disk, and
control means for controlling said disk rotating means and said sample analysis means.

2. An analysis apparatus according to claim 1, wherein a plurality of said sample development surfaces are provided, each of said plurality of surfaces being divided into compartments reaching from a center to a circumference of said disk.

3. An analysis apparatus according to claim 1 or 2, wherein said optical measuring head is constructed to expose a convergent light on said sample.

4. An analysis apparatus according to claim 1 or 2, wherein said optical measuring head is positioned at a side opposite to said sample development surfaces, to expose a polarized laser convergent light on said samples.

5. An analysis apparatus according to claim 1 or 2, wherein said optical measuring head is constructed to expose a linear pattern light having a length in a radial direction of said disk, on said sample development surfaces .

# F I G. 1

# F I G. 2 a

# F I G. 2 b

# F I G. 3

# F I G. 4

## F I G. 5 a

## F I G. 5 b

# F I G. 6

# F I G. 7